# EUROPEAN PATENT SPECIFICATION

(11) **EP 2 308 409 B1**
(45) Date of publication and mention of the grant of the patent: **26.03.2014**
(21) Application number: 09803105.7
(22) Date of filing: 06.07.2009
(51) Int. Cl.: A61C 3/02, A61C 1/05, A61C 1/08, A61B 17/16, A61C 8/00, B23B 49/00, B23B 51/04

(54) **DRILL FOR IMPLANT**
BOHRER FÜR EIN IMPLANTAT
FORET POUR IMPLANT

(30) Priority: 29.07.2008 KR 20080074040
(43) Date of publication of application: 13.04.2011
(73) Proprietor: Megagen Implant Co., Ltd., Gyeongsangbuk-do 712-852 (KR)
(72) Inventor: LEE, Samuel Soon Ho, Buena Park, CA 90621 (US)
(74) Representative: Grünecker, Kinkeldey, Stockmair & Schwanhäusser
(86) International application number: PCT/KR2009/003679
(87) International publication number: WO 2010/013900

(56) References cited:
- WO-A1-2008/088105
- WO-A2-2004/100820
- FR-A- 1 027 829
- KR-B1- 100 619 145
- KR-B1- 100 759 261
- KR-Y1- 200 338 095
- US-A- 4 741 651
- US-A- 4 820 156

## Description

### [TECHNICAL FIELD]

### [BACKGROUND ART]

The inventive concept relates to a trephine drill for an implant, which can not only precisely perforate an alveolar bone at a preset depth, but also easily remove an alveolar bone piece, generated when perforating the alveolar bone, from inside of a perforating body. An implant is a replacement to support restorations and act as a missing biological structure. In particular, a dental implant is an artificial tooth transplant, in which an artificial tooth root made of titanium or the like that is not rejected by a human body is implanted in a toothless alveolar bone in order to replace a missing tooth root, and then an artificial tooth is settled to restore a tooth function.

While a general prosthesis or denture injures neighboring teeth and bones as time goes by, the dental implant does not injure neighboring tooth tissue and has the same function or shape as an original tooth without causing any cavity, so that it can be semipermanently used.

Also, the dental implant promotes the function of the artificial tooth with regard to partial and total anodontia patients as well as restoration of a single missing tooth, and improves an aesthetic aspect of prosthetic restoration. Further, the dental implant distributes excessive stress applied to neighboring supporting-bone tissue and assists in stabilizing a teeth row.

Meanwhile, because the alveolar bone is perforated by a drill or the like and then the implant is placed into a perforated part, a perforating operation for the alveolar bone is very important.

For example, the perforating operation of the drill for implanting the implant will be schematically described as follows. The following perforating operation is just exemplified and not limited thereto. Alternatively, various perforating operations may be possible.

To begin with, a round drill is used as the first use drill and determines a placement position of the implant on the surface of the alveolar bone.

Then, an upper end part of the alveolar bone is cut and a little opened in a part where the teeth are lost, and then a guide drill is mounted to a predetermined tool, thereby forming a predetermined-deep hole in the alveolar bone while supplying a saline solution thereto. Next, a first drill, a final drill, etc. are employed to enlarge the hole formed by the guide drill. In other words, a plurality of trephine drills is used to form a hole corresponding to the width and length of a fixture.

Then, a tap drill is used to form a screw thread on an inner wall of the hole in the alveolar bone.

After placing the fixture into the hole having the screw thread in the alveolar bone, an abutment is fastened to the fixture and an artificial tooth is fixed to the abutment by an adhesive.

However, although the perforating operation has to be precisely performed at a perforating depth preset in consideration of the state, bone density, etc. of the alveolar bone when the alveolar bone is perforated by the above method, it is conventionally difficult to precisely control the perforating depth of the alveolar bone. Further, since a bone piece, generated when perforating the alveolar bone, i.e., an alveolar bone piece is inserted in the inside of the drill, there are difficulties in removing the alveolar bone piece, for example, an separate tool is needed to remove such an alveolar bone piece.

Accordingly, there is a need of developing a trephine drill for an implant, which can not only perform a preset-deep perforating operation for the alveolar bone where the implant will be placed, but also easily remove a bone piece, which remains in the drill after completing the perforating operation, i.e., an alveolar bone piece.

WO 2008/088105 A1 discloses a drill for operating implant. The drill comprises a drill body which is securely connected to a shank. Further, a stopper is mounted inside the drill body to limit a depth of a drill hole drilled in the alveolar bone.

### [DISCLOSURE]

### [TECHNICAL PROBLEM]

The inventive concept provides a trephine drill for an implant, which can not only precisely perforate an alveolar bone at a preset depth, but also easily remove an alveolar bone piece, generated when perforating the alveolar bone, from inside of a perforating body.

With this, not only an alveolar bone is precisely perforated at a preset depth, but also an alveolar bone piece, generated when perforating the alveolar bone, is easily removed from inside of a perforating body.

### [ADVATAGEOUS EFFECTS]

With this, not only an alveolar bone is precisely perforated at a preset depth, but also an alveolar bone piece, generated when perforating the alveolar bone, is easily removed from inside of a perforating body.

### [BEST MODE]

According to an aspect of the inventive concept, there is provided a trephine drill, in which a sinus membrane inside an alveolar bone is lifted after the alveolar bone is perforated for a placement of an implant, the drill tool including: a perforating drill to perforate the alveolar bone up to a part adjacent to the sinus membrane, the perforating drill including: a perforating body which includes a first end part provided with a cutter for perforating the alveolar bone and a second end part formed with a through hole; and a perforating rod which couples with the perforating body to move close to and apart from the first end part of the perforating body toward the through hole of the perforating body and adjusts a perforation depth when perforating the alveolar bone.

The inventive trephine drill further includes a fastening unit coupled to the perforating body to move close to and apart from the perforating rod in a direction transverse to an axis of the perforating body and settles a relative position of the perforating rod with regard to the perforating body.

The trephine drill may further include an installation hole penetrating through a lateral wall of the perforating body along a thickness direction, wherein the fastening unit is coupled to the perforating body through the installation hole to move close to and apart from the perforating rod.

The installation hole may be internally formed with a female-screw thread, and the fastening unit may be a fastening screw externally formed with a male-screw thread corresponding to the female-screw thread.

A head part of the fastening screw facing the perforating rod may be grooved to have a wrench socket to which a wrench is detachably coupled for rotating the fastening screw in forward and backward directions to move the fastening screw close to and apart from the perforating rod, respectively.

The fastening unit may be made of either of titanium or stainless steel, and the perforating rod may be made of stainless steel.

According to the invention, the perforating rod is formed with a male-screw portion on at least a part of a periphery thereof, and the through hole of the perforating body is formed with a female-screw portion on an inner wall thereof corresponding to the male-screw portion.

The periphery of the perforating rod, formed with the male-screw portion along a lengthwise direction of the perforating rod, may be provided with a non-screw portion formed by cutting.

The non-screw portion may be either of a flat portion provided flatly along the lengthwise direction of the perforating rod or a groove portion recessed inward at a predetermined depth along the lengthwise direction of the perforating rod.

The perforating body may further include an accommodating portion in a central region thereof to accommodate bone bits of the alveolar bone when perforating the alveolar bone, and a discharge hole penetrating a lateral wall thereof in a thickness direction, communicating with the accommodating portion and discharging the bone bits of the alveolar bone accommodated in the accommodating portion.

The perforating rod may further include a connecting groove at one end part thereof to which a hand piece that provides a rotational force is connected, and a through hole penetrating an inside thereof in an axial direction to supply a saline solution to an operative part when the perforating body rotates and perforates the alveolar bone.

The perforating body may further include a catching unit on an inner wall thereof to catch an alveolar bone piece generated when perforating the alveolar bone.

The catching unit may be a ratchet-type female screw to catch the alveolar bone only if the perforating body rotates in one direction.

### [DESCRIPTION OF DRAWINGS]

FIG. 1 is a perspective view of a trephine drill for an implant according to an exemplary embodiment of the present invention
FIG. 2 is an exploded perspective view of FIG. 1;
FIG. 3 is a view for explaining a process that the alveolar bone is perforated by the trephine drill of FIG. 1 for an implant;
FIG. 4 is a vertical sectional view of FIG. 1;
FIG. 5 is a perspective view of a rod body provided with a flat portion;
FIG. 6 is a perspective view of a rod body provided with a groove portion; and
FIG. 7 is a perspective view of a trephine drill for an implant according to another exemplary embodiment of the present invention.

### [MODE FOR INVENTION]

The attached drawings for illustrating embodiments of the inventive concept are referred to in order to gain a sufficient understanding of the inventive concept and the merits thereof. Hereinafter, the inventive concept will be described in detail by explaining embodiments of the inventive concept with reference to the attached drawings. Like reference numerals in the drawings denote like elements.

First, drills used in perforating an alveolar bone will be schematically described before describing a trephine drill for an implant according to an exemplary embodiment of the present invention.

As the drills used in perforating the alveolar bone, there are a round drill used as an initial drill to point out a position of the alveolar bone where an implant is placed; a guide drill primarily carrying out a perforating operation; a trephine drill for an implant according to an exemplary embodiment of the present invention, which is inserted in a hole of the alveolar bone perforated by the guide drill and carries out a perforating operation up to a preset depth; a tap drill forming a screw thread on an inner wall of the hole in the alveolar bone perforated by the trephine drill for the implant, the screw thread being provided for installation of a fixture; etc.

By these drills, the hole for the placement of the implant may be formed, so that the fixture can be firmly fastened to the hole of the alveolar bone. However, during the perforating operation, particularly, when carrying out the perforating operation by the trephine drill for the implant, the hole has to be precisely perforated corresponding to the length of the fixture, and a bone piece generated during the perforating operation, i.e., an alveolar bone piece has to be easily removed from the inside of the drill.

To this end, the trephine drill for the implant according to an exemplary embodiment of the present invention will be described below in more detail. For example, this embodiment will illustrate a case that the trephine drill for the implant perforates the alveolar bone positioned in a lower jaw, but not limited thereto. Alternatively, it will be appreciated that the trephine drill for the implant according to this embodiment may be used in perforating the alveolar bone in an upper jaw or the other positions.

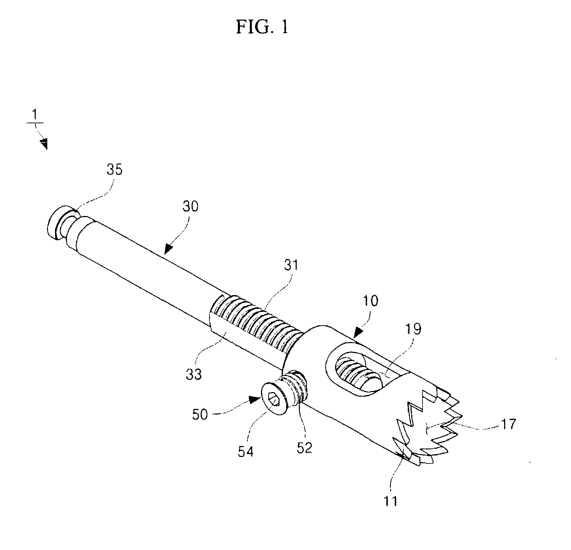

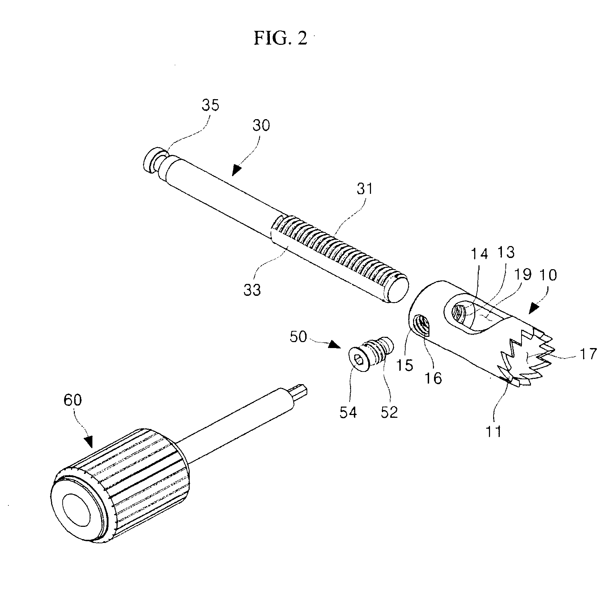

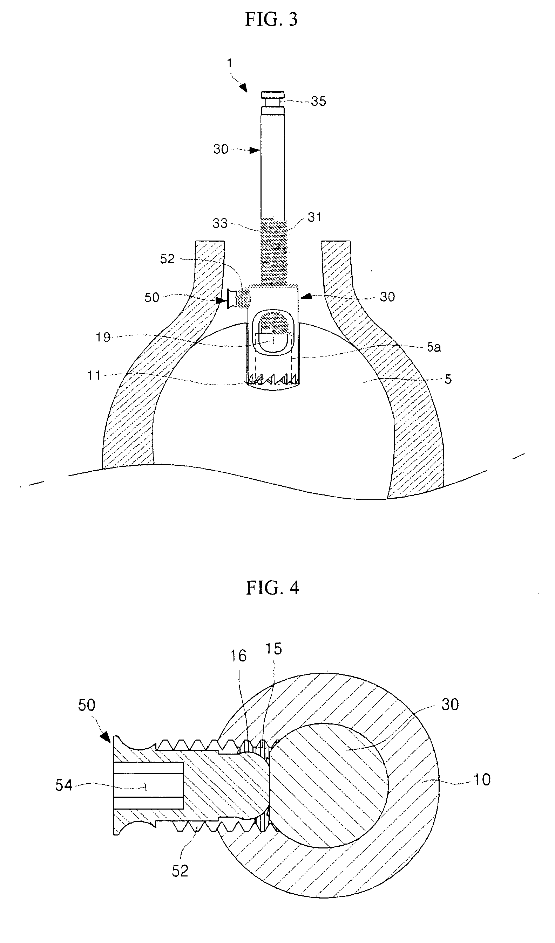

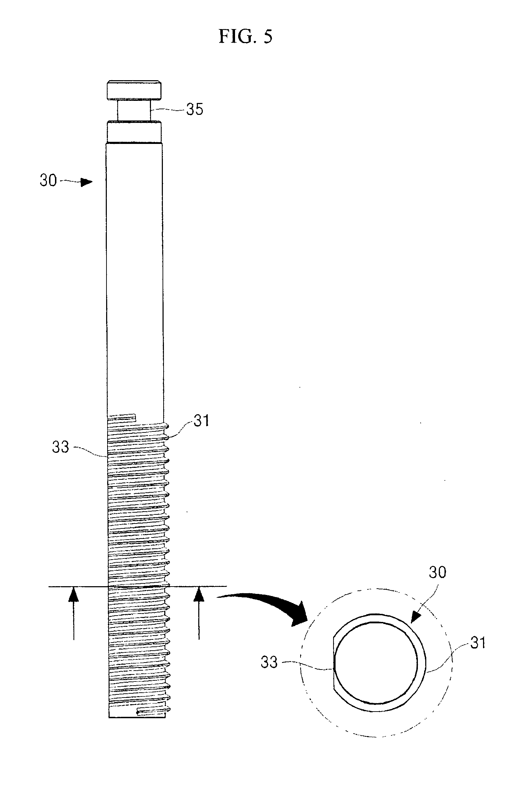

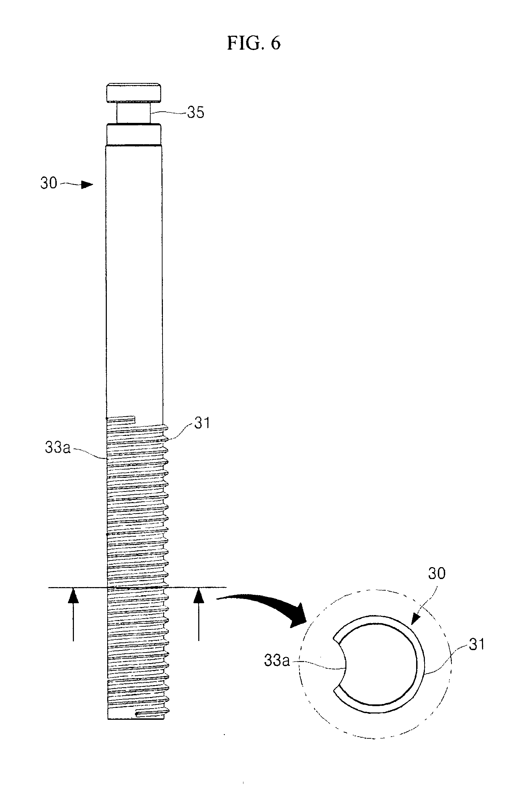

FIG. 1 is a perspective view of a trephine drill for an implant according to an exemplary embodiment of the present invention, FIG. 2 is an exploded perspective view of FIG. 1, FIG. 3 is a view for explaining a process that the alveolar bone is perforated by the trephine drill of FIG. 1 for an implant, FIG. 4 is a vertical sectional view of FIG. 1, FIG. 5 is a perspective view of a rod body provided with a flat portion, and FIG. 6 is a perspective view of a rod body provided with a groove portion.

As shown therein, a trephine drill 1 according to this embodiment is provided as a drill for perforating an alveolar bone 5 (see FIG. 3) positioned in an upper jaw, a lower jaw, or the like, which includes a perforating body 10 shaped like a hollow cylinder and having a first end part facing toward the alveolar bone 5 and formed with cutters 11 for perforating the alveolar bone 5 and a second end part formed with a through hole 13; a rod body 30 coupled to the through hole 13 of the perforating body 10 movably along the inside of the perforating body 10 and adjusting a perforation depth of the alveolar bone 5; and a fastening unit 50 coupled to the perforating body 10 movably along a thickness direction of a lateral wall of the perforating body 10 and fastening the position of the rod body 30 with regard to the perforating body 10.

As shown in FIGs. 1 and 2, the perforating body 10 is shaped like a top-opened hollow cylinder, and includes the serrated cutters 11 at an upper end part thereof and the through hole 13 at a lower end part thereof.

Further, the perforating body 10 is formed with an accommodating portion 17 in a central region thereof along a depth direction to accommodate bone bits 5a, i.e., alveolar bone pieces 5a (refer to FIG. 3) generated when perforating the alveolar bone 5. Also, the perforating body 10 is formed with a discharge hole 19 on a lateral wall thereof to communicate with the accommodating portion 17 and easily discharge the alveolar bone pieces 5a produced from the alveolar bone 5 when perforating the alveolar bone 5 to the outside. In this embodiment, the alveolar bone pieces 5a generated when perforating the alveolar bone 5 are removed through the discharge hole 19, but not limited thereto. Alternatively, the alveolar bone pieces 5a may be removed by a separate instrument (not shown) through the accommodating portion 17.

The perforating body 10 rotates simultaneously with the rod body 30 and goes ahead toward the inside of the alveolar bone 5 when the rod body 30 to be described later is rotated by a rotational force provided by a hand piece (not shown), thereby forming a hole on the alveolar bone 5 for the placement of the implant (not shown).

For example, if the thickness of the alveolar bone 5 to be perforated is about 5mm, i.e., if a fixture (not shown) to be embedded has a length of about 5mm, the alveolar bone 5 is perforated to a depth of about 2mm by the foregoing guide drill (not shown), and perforated to a depth of about 5mm from the surface of the alveolar bone 5 by the cutters 11 of the perforating body 10 in this embodiment.

Accordingly, to ascertain the thickness of the alveolar bone 5 and the state of a sinus membrane 3 before perforating the alveolar bone 5, an X-ray or the like may be previously taken. In this case, the perforation depth of each drill has to be determined on the basis of taken information.

However, according to a conventional drill (not shown), it is difficult to perforate to a desired depth, and thus the perforation is likely to be implemented more or less than a depth to be perforated. Therefore, it is troublesome to do the perforation again.

To solve this problem, the trephine drill 1 for the implant in this embodiment includes the rod body 30 movable along a lengthwise direction inside the perforating body 10 to perforate to a desired depth.

As shown in FIGs. 1 and 2, the rod body 30 in this embodiment is movable along the longitudinal direction inside the perforating body 10 relative to the perforating body 10 so as to easily adjust the position with respect to the perforating body 10, and settled by the fastening unit 50 to be described later to thereby carry out the perforation firmly when perforating the alveolar bone 5.

The rod body 30 allows the perforating body 10 to go ahead by only a predetermined depth, i.e., a depth previously set by the rod body 30, so that the hole corresponding to the length of the fixture can be formed in the alveolar bone 5.

Specifically, the rod body 30 moves relative to the perforating body 10 and is then settled so that a distance from a front end part of the rod body 30 to a front end part of the perforating body 10, i.e., to an upper end part of the cutter 11 can be about 2mm. Then, if the alveolar bone 5 is perforated in this state, the alveolar bone 5 is perforated to a depth of 2mm by the cutters 11 of the perforating body 10. If the alveolar bone 5 to be perforated is relatively thick, e.g., has a thickness of 3mm, the rod body 30 moves relative to the perforating body 10 and is then settled to the perforating body 10 so that a distance from the front end part of the rod body 30 to the front end part of the perforating body 10 can be 3mm. Then, if the alveolar bone 5 is perforated in this state, the alveolar bone 5 is perforated as much as 3mm.

In other words, since the rod body 30 is relatively movable with respect to the perforating body 10, the position of the rod body 30 is adjustable with respect to the perforating body 10 according to the preset perforation depth. Further, the perforation is carried out after fastening the rod body 30 with respect to the perforating body 10, so that the perforation can be easily and firmly implemented as much as a previously set depth. To this end, as shown in FIG. 2, the rod body 30 is formed with a male-screw portion 31 on the periphery thereof, so that the rod body 30 can move forward, i.e., in an inward direction of the perforating body 10 or backward, i.e., in an opposite direction. Likewise, the through hole 13 of the second end part of the perforating body 10 is formed with a female-screw portion 14 corresponding to the male-screw portion 31 of the rod body 30.

Thus, if the rod body 30 is rotated in a forward direction, the rod body 30 moves forward to the inside of the perforating body 10. On the other hand, if the rod body 30 is rotated in a backward direction, the rod body 30 moves backward from the perforating body 10. With this, the position of the rod body 30 can be easily adjusted with respect to the perforating body 10.

Additionally, the rod body 30 should not move forward or backward or rotate with respect to the perforating body 10 while perforating the alveolar bone 5. If the rod body 30 is not fixed to the perforating body 10 during the perforation, the alveolar bone 5 may be perforated to an inaccurate depth. For example, if the rod body 30 moves forward with respect to the perforating body 10 during the perforation, a hole may be more shallowly formed on the alveolar bone 5 than a desired depth. If the rod body 30 moves backward with respect to the perforating body 10 during the perforation, a hole may be more deeply formed on the alveolar bone 5 than a desired depth.

Therefore, in order to fasten the rod body 30, the fastening unit 50 is movably coupled to the perforating body 10.

In the present embodiment, the fastening unit 50 is provided as a fastening screw 50 having a male-screw thread 52 so that it can rotate to easily fasten the rod body 30 to the perforating body 10.

As shown in FIG. 4, the fastening screw 50 is detachably coupled to an installation hole 15 penetrating through the lateral wall of the perforating body 10 so that it can move close to and apart from the rod body 30 along the thickness direction of the lateral wall of the perforating body 10, i.e., in a direction transverse to an axis of the rod body 30. The fastening screw 50 may be either made of titanium or stainless steel.

The installation hole 15 of the perforating body 10 has a female-screw thread 16 corresponding to the male-screw thread 52 of the fastening screw 50. Thus, if the fastening screw 50 is rotated in a forward direction, the fastening screw 50 moves forward and presses one side of the rod body 30, i.e., a flat portion 33 to be described later, thereby fastening the rod body 30 in the perforating body 10. On the other hand, if the fastening screw 50 is rotated in a backward direction, the fastening screw 50 moves backward and releases the rod body 30, thereby allowing the rod body 30 to be movable with regard to the perforating body 10.

In the case that the fastening unit 50 is in contact with the rod body 30, the rod body 30 has a non-screw portion 33, e.g., a flat portion 33 in this embodiment to effectively transfer a pressing force. The flat portion 33 is formed by cutting a predetermined region of the male-screw portion 31. Thus, when the fastening screw 50 presses the flat portion 33 of the rod body 30, the rod body 30 does not move relative to the perforating body 10, so that the perforation can be firmly achieved with respect to the alveolar bone 5.

In the foregoing embodiment, the flat portion 33 (refer to FIG. 5) is employed as the non-screw portion 33 of the rod body 30 in order to prevent the rod body 30 from moving relative to the perforating body 10, but not limited thereto. Alternatively, if possible, not the flat portion 33 but a groove portion 33a (refer to FIG. 6) may be used as the non-screw portion 33a of the rod body 30. The groove portion 33a receives one end part of the fastening screw 50 by a predetermined depth, thereby strengthening coupling between the rod body 30 and the perforating body 10.

As shown in FIG. 2, the fastening screw 50 may be fastened to or released from the perforating body 10 by a hex wrench 60. Further, a head part of the fastening screw 50 is formed with a hex socket 54 recessed in the form of a hexagon, so that the hex wrench 60 can be detachably inserted in the hex socket 54 to rotate the fastening screw 50 in the forward and backward directions. Thus, the fastening screw 50 can move forward, i.e., toward the inside of the perforating body 10 and backward, i.e., toward the outside of the perforating body 10.

Since the rod body 30 is movable along the lengthwise direction of the perforating body 10 inside the perforating body 10, the alveolar bone pieces 5a (refer to FIG. 3) that may remain inside the perforating body 10 is more easily removed than a conventional one. That is, if the trephine drill 1 for the implant in this embodiment is separated from the alveolar bone 5 after the perforating body 10 rotates to perforate the alveolar bone 5, the alveolar bone pieces 5a cut by the cutters 11 remain inside the perforating body 10.

Here, the alveolar bone pieces 5a has to be removed from the perforating body 10 in order to do the perforation again. Conventionally, there has been used a method or the like that removes the alveolar bone pieces 5a by inserting a separate instrument (not shown). On the contrary, in this embodiment, the rod body 30 pushes the alveolar bone pieces 5a out while moving toward the inside of the perforating body 10, so that the residual alveolar bone pieces 5a can be easily removed without a separate instrument.

Meanwhile, the rod body 30 is provided with a connecting groove 35 at the second end part thereof to which a hand piece for providing a rotational force is detachably coupled, so that the alveolar bone 5 can be perforated by the rotational force of the hand piece. Further, the rod body 30 may be internally formed with a through hole (not shown) that communicates with an inner space of the perforating body 10. In the case that the through hole is formed in the rod body 30, it is possible to supply a saline solution via the through hole, thereby making the perforation smooth.

By the trephine drill 1 with the above configuration, an operation of perforating the alveolar bone 5 will be described as follows.

First, the state of the alveolar bone 5 to be perforated is inspected by the X-ray or the like, and the perforation depth is then determined for the alveolar bone 5.

Then, the round drill (not shown) is used as the initial drill to point out a placement position for the implant on the surface of the alveolar bone 5. Then, an upper end part of the alveolar bone 5 is cut and a little opened in a part where the teeth are lost, and then the guide drill is used to form a predetermined-deep shallow hole. If the determined perforation-depth for the alveolar bone 5 is about 5mm, the guide drill forms the shallow hole having a depth of about 2mm on the alveolar bone 5.

Then, as shown in FIG. 3, the trephine drill 1 for the implant in this embodiment is used to perforate up to a preset depth, e.g., up to a depth of about 5mm from the surface of the alveolar bone 5. At this time, because the alveolar bone 5 has already been perforated by the guide drill up to a depth of 2mm from the surface thereof, the trephine drill 1 has to perforate to an inward depth of 3mm from the bottom of the hole of the alveolar bone 5 perforated by the guide drill 100. To this end, the position of the rod body 30 relative to the perforating body 10 is adjusted depending on the preset depth.

Such an adjusting process can be achieved as follows. First, the fastening unit 50, i.e., the fastening screw 50 in this embodiment is rotated to thereby release the fastening screw 50 from the perforating body 10. Then, the position of the rod body 30 relative to the perforating body 10 is adjusted by rotating the rod body 30 in the forward or backward direction so that a height difference between the front end part of the perforating body 10 and the front end part of the rod body 30 can be 3mm. Then, the fastening screw 50 is inserted again in the installation hole 15 of the perforating body 10, and rotated so that the front end part of the fastening screw 50 can press the flat portion 33 of the rod body 30.

Then, the trephine drill 1 is placed in a part of the alveolar bone 5 to be perforated, and then carries out the perforation. After the perforation is completed, the rod body 30 is rotated to move forward in an upper direction of the perforating body 10 so as to remove the alveolar bone pieces 5a remained in the perforating body 10.

Next, the tap drill (not shown) is used to form a screw thread (not shown) on an inner wall of the hole in the alveolar bone 5, the hole perforated by the trephine drill 1 for the implant. Then, a fixture (not shown) is placed into the hole having the screw thread in the alveolar bone and undergoes osseointegration for a predetermined period of time. Then, an abutment (not shown) is fastened to the fixture and an artificial tooth (not shown) is fixed to the abutment, thereby completing an implant operation.

According to an exemplary embodiment of the present invention, the alveolar bone 5 is perforated as much as to a preset depth, so that not only the perforating operation can be precisely achieved without conventional troublesome of doing the perforation again, but also the alveolar bone piece 5a that may remain inside the perforating body 10 after the perforating operation can be easily removed without any additional tool.

Meanwhile, a trephine drill for an implant according to another exemplary embodiment of the present invention will be described below with reference to an accompanying drawing. However, repetitive description with respect to the foregoing embodiment will be avoided as necessary.

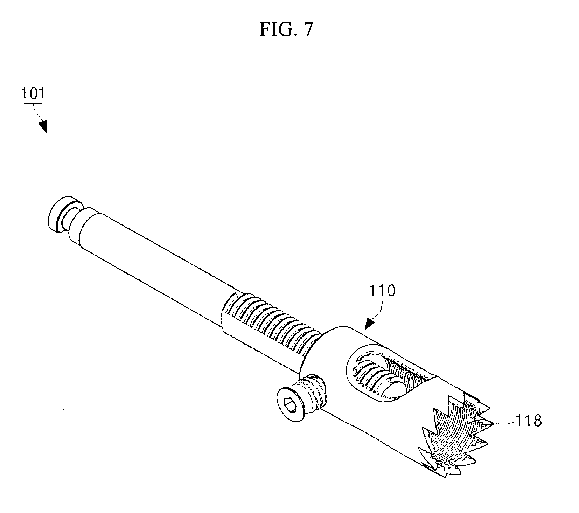

FIG. 7 is a perspective view of a trephine drill for an implant according to another exemplary embodiment of the present invention. As shown therein, a trephine drill 101 for an implant according to this embodiment includes a catching unit 118 on an inner wall thereof as a ratchet-type female screw.

The catching unit 118 catches bone bits, i.e., alveolar bone pieces generated while perforating an alveolar bone (not shown), so that the alveolar bone pieces cut from the alveolar bone can be easily removed out.

As described above, since the catching unit 118 is provided as the ratchet-type female screw, the cut bone bits, i.e., the alveolar bone pieces are drawn and caught inside a perforating body 110 only when the perforating body 110 rotates in a forward direction. On the other hand, when the perforating body 110 rotates in a backward direction, the alveolar bone pieces are removed as being caught in the catching unit 118, so that the alveolar bone pieces generated during the perforation can be easily removed through the hole of the alveolar bone.

According to another exemplary embodiment of the present invention, the bone bits, i.e., the alveolar bone pieces generated when perforating the alveolar bone are easily discharged out from the inside of the hole of the alveolar bone, so that there is no additional following job for removing the alveolar bone pieces, thereby reducing a time taken in the surgical operation.

In the foregoing embodiment, the rod body formed with the male-screw portion on the periphery thereof, but not limited thereto. Alternatively, not the male-screw portion but other configurations may be provided to adjust the position of the rod body with respect to the perforating body. Also, in the foregoing embodiment, the fastening unit has a screw form such as a fastening screw, but not limited thereto. Alternatively, other configurations may be possible as long as it can press the rod body with respect to the perforating body.

As described above, according to the present inventive concept, a trephine drill for an implant can not only precisely perforate an alveolar bone at a preset depth, but also easily remove an alveolar bone piece, generated when perforating the alveolar bone, from inside of a perforating body.

### [INDUSTRIAL APPLICABILITY]

According to the present inventive concept, not only an alveolar bone is precisely perforated at a preset depth, but also an alveolar bone piece, generated when perforating the alveolar bone, is easily removed from inside of a perforating body.

## Claims

1. A trephine drill (1) for an implant, comprising:
a perforating body (10) which comprises a first end part provided with a cutter (11) for perforating an alveolar bone (5) and a second end part; and
a rod body (30) which couples with the perforating body (10),
**characterized in**
**that** the second end part of the perforating body (10) comprises a through hole (13), wherein the rod body (30) is coupled to the through hole (13) and is adapted to move close to and apart from the first end part of the perforating body (10) along an inside of the perforating body (10), and is further adapted to adjust a perforation depth when perforating the alveolar bone (5),
**that** the trephine drill (1) further comprises a fastening unit (50) coupled to the perforating body (10) to move close to and apart from the rod body (30) in a direction transverse to an axis of the perforating body (10) and settles a relative position of the rod body (30) with regard to the perforating body (10), and
**that** the rod body (30) is formed with a male-screw portion (31) on at least a part of a periphery thereof, and
the through hole (13) of the perforating body (10) is formed with a female-screw portion (14) on an inner wall thereof corresponding to the male-screw portion (31).

2. The trephine drill according to claim 1, further comprising an installation hole(15) penetrating through a lateral wall of the perforating body (10) along a thickness direction, wherein the fastening unit (50) is coupled to the perforating body (10) through the installation hole (15) to move close to and apart from the rod body (30).

3. The trephine drill according to claim 2, wherein the installation hole (15) is internally formed with a female-screw thread (16), and
the fastening unit (50) is a fastening screw externally formed with a male-screw thread (52) corresponding to the female-screw thread (16).

4. The trephine drill according to claim 3, wherein a head part of the fastening screw facing the rod body (30) is grooved to have a wrench socket to which a wrench is detachably coupled.

5. The trephine drill according to one of the claims 1 to 4, wherein the fastening unit (50) is made of either of titanium or stainless steel, and
the rod body (30) is made of stainless steel.

6. The trephine drill according to one of the previous claims, wherein the periphery of the rod body (30), formed with the male-screw portion (31) along a lengthwise direction of the rod body (30), is provided with a non-screw portion (33) formed by cutting.

7. The trephine drill according to claim 6, wherein the non-screw portion (33) is either of a flat portion provided flatly along the lengthwise direction of the rod body (30) or a groove portion (33a) recessed inward at a predetermined depth along the lengthwise direction of the rod body (30).

8. The trephine drill according to one of the previous claims , wherein the perforating body (10) further comprises an accommodating portion (17) in a central region thereof to accommodate bone bits (5a) of the alveolar bone (5) when perforating the alveolar bone (5), and a discharge hole (19) penetrating a lateral wall thereof in a thickness direction, communicating with the accommodating portion (17) and discharging the bone bits (5a) of the alveolar bone (5) accommodated in the accommodating portion (17).

9. The trephine drill according to one of the previous claims, wherein the rod body (30) further comprises a connecting groove (35) at one end part thereof to which a hand piece that provides a rotational force is connected, and a supply through hole penetrating an inside of the rod body (30) in an axial direction to supply a saline solution to an operative part when the perforating body (10) rotates and perforates the alveolar bone (5).

10. The trephine drill according to one of the previous claims, wherein the perforating body (10) further comprises a catching unit (118) on an inner wall thereof to catch an alveolar bone piece (5a) generated when perforating the alveolar bone (5).

11. The trephine drill according to claim 10, wherein the catching unit (118) is a ratchet-type female screw to catch the alveolar bone pieces (5a) only if the perforating body (10) rotates in one direction.

## Patentansprüche

1. Trepanbohrer (1) für ein Implantat, umfassend:
einen Perforierkörper (10), der einen ersten Endteil mit einem Fräser (11) zum Perforieren eines Alveolarknochens (5) und einen zweiten Endteil umfasst; und
einen Stabkörper (30), der mit dem Perforierkörper (10) verbunden wird,
**dadurch gekennzeichnet,**
**dass** der zweite Endteil des Perforierkörpers (10) eine Durchgangsbohrung (13) aufweist, wobei der Stabkörper (30) durch die Durchgangsbohrung (13) verbunden und dazu eingerichtet ist, sich entlang einer Innenseite des Perforierkörpers (10) auf den ersten Endteil zu und von diesem weg zu bewegen und des Weiteren dazu eingerichtet ist, beim Perforieren des Alveolarknochens (5) eine Perforiertiefe einzustellen,
**dass** der Trepanbohrer (1) des Weiteren eine Befestigungseinheit (50) umfasst,
die so mit dem Perforierkörper (10) verbunden ist, dass sie sich in einer Richtung quer zu einer Achse des Perforierkörpers (10) auf den Stabkörper (30) zu und von diesem weg bewegt und eine relative Position des Stabkörpers (30) bezüglich des Perforierkörpers (10) festlegt, und
**dass** der Stabkörper (30) mit einem Außengewindeteil (31) an zumindest einem Teil seines Umfangs ausgebildet ist, und
die Durchgangsbohrung (13) des Perforierkörpers (10) mit einem Innengewindeteil (14) an ihrer Innenwand ausgebildet ist, der mit dem Außengewindeteil (31) korrespondiert.

2. Trepanbohrer nach Anspruch 1, des Weiteren umfassend eine Montagebohrung (15), die eine Seitenwand des Perforierkörpers (10) entlang einer Dickenrichtung durchdringt, wobei die Befestigungseinheit (50) durch die Montagebohrung (15) an dem Perforierkörper (10) befestigt ist, um sich auf den Stabkörper (30) zu und von diesem weg zu bewegen.

3. Trepanbohrer nach Anspruch 2, wobei die Montagebohrung (15) mit einem Innengewinde (16) an der Innenseite ausgebildet ist, und
die Befestigungseinheit (50) eine Befestigungsschraube mit einem externen Außengewinde (52) ist, das mit dem Innengewinde (16) korrespondiert.

4. Trepanbohrer nach Anspruch 3, wobei ein Kopfteil der Befestigungsschraube gegenüber dem Stabkörper (30) genutet ist und eine Steckschlüsselaufnahme aufweist, mit der lösbar ein Steckschlüssel verbunden werden kann.

5. Trepanbohrer nach einem der Ansprüche 1 bis 4, wobei die Befestigungseinheit (50) entweder aus Titan oder nichtrostendem Stahl besteht, und
der Stabkörper (30) aus nichtrostendem Stahl besteht.

6. Trepanbohrer nach einem der vorhergehenden Ansprüche, wobei der Umfang des Stabkörpers (30), der über das Außengewinde (31) entlang einer Längsrichtung des Stabkörpers (30) verfügt, mit einem gewindefreien Teil (33) versehen ist, der durch Zerspanen gebildet wird.

7. Trepanbohrer nach Anspruch 6, wobei der gewindefreie Teil (33) entweder ein flacher Abschnitt ist, der entlang der Längsrichtung des Stabkörpers (30) ausgebildet ist, oder ein genuteter Abschnitt (33a) ist, der um eine vorgegebene Tiefe entlang der Längsrichtung des Stabkörpers (30) nach innen vertieft ist.

8. Trepanbohrer nach einem der vorhergehenden Ansprüche, wobei der Perforierkörper (10) des Weiteren einen Aufnahmeabschnitt (17) in einem mittleren Bereich aufweist, um Knochenstückchen (5a) des Alveolarknochens (5) beim Perforieren des Alveolarknochens (5) aufzunehmen, und eine Entleerungsöffnung (19), die eine Seitenwand in deren Dickenrichtung durchdringt, mit dem Aufnahmeabschnitt (17) in Verbindung steht und die in dem Aufnahmeabschnitt (17) aufgenommenen Knochenstückchen (5a) des Alveolarknochens (5) entlädt.

9. Trepanbohrer nach einem der vorhergehenden Ansprüche, wobei der Stabkörper (30) des Weiteren an einem Ende eine Verbindungsnut (35) aufweist, mit der ein Handstück, das eine Drehkraft überträgt, verbunden wird, und eine Versorgungsdurchgangsbohrung, die eine Innenseite des Stabkörpers (30) in axialer Richtung durchdringt, um einem operativen Teil eine Salzlösung zuzuführen, wenn sich der Perforierkörper (10) dreht und den Alveolarknochen (5) perforiert.

10. Trepanbohrer nach einem der vorhergehenden Ansprüche, wobei der Perforierkörper (10) des Weiteren eine Auffangeinheit (118) an einer Innenwand desselben umfasst, um ein Alveolarknochenstück (5a), das beim Perforieren des Alveolarknochens (5) entsteht, aufzufangen.

11. Trepanbohrer nach Anspruch 10, wobei die Auffangeinheit (118) eine ratschenartige Schraube mit Innengewinde ist, welche die Alveolarknochenstücke (5a) nur in einer Drehrichtung des Perforierkörpers (10) aufnimmt.

## Revendications

1. Trépan (1) pour un implant, comprenant :
un corps perforant (10) qui comprend une première partie terminale pourvue d'un couteau (11) pour perforer un os alvéolaire (5) et une deuxième partie terminale ; et
un corps de tige (30) qui s'accouple au corps perforant (10),
**caractérisé en ce que**
la deuxième partie terminale du corps perforant (10) comprend un trou traversant (13), dans lequel le corps de tige (30) est accouplé au trou traversant (13) et est adapté pour se déplacer à proximité et à distance de la première partie terminale du corps perforant (10) le long d'une partie intérieure du corps perforant (10), et est en outre adapté pour ajuster une profondeur de perforation lors de la perforation de l'os alvéolaire (5),
le trépan (1) comprend en outre une unité de fixation (50) accouplée au corps perforant (10) pour se déplacer à proximité et à distance du corps de tige (30) selon une direction transversale à un axe du corps perforant (10) et détermine une position relative du corps de tige (30) par rapport au corps perforant (10),
et
le corps de tige (30) est formé avec une section de vis mâle (31) sur au moins une partie d'une périphérie de celle-ci, et
le trou traversant (13) du corps perforant (10) est formé avec une section de vis femelle (14) sur une paroi interne de celui-ci correspondant à la section de vis mâle (31).

2. Trépan selon la revendication 1, comprenant en outre un trou d'installation (15) qui pénètre une paroi latérale du corps perforant (10) en direction de l'épaisseur, dans lequel l'unité de fixation (50) est accouplée au corps perforant (10) à travers le trou d'installation (15) pour se déplacer à proximité et à distance du corps de tige (30).

3. Trépan selon la revendication 2, dans lequel
le trou d'installation (15) est formé avec un filetage femelle interne (16), et
l'unité de fixation (50) est une vis de fixation formée avec un filetage mâle externe (52) correspondant au filetage femelle (16).

4. Trépan selon la revendication 3, dans lequel une partie de tête de la vis de fixation qui fait face au corps de tige (30) est rainurée pour recevoir une douille de clé à laquelle une clé est accouplée de manière amovible.

5. Trépan selon l'une des revendications 1 à 4, dans lequel
l'unité de fixation (50) est constituée soit de titane, soit d'acier inoxydable, et
le corps de tige (30) est constitué d'acier inoxydable.

6. Trépan selon l'une des revendications précédentes, dans lequel la périphérie du corps de tige (30), formée avec la section de vis mâle (31) selon une direction longitudinale du corps de tige (30), est pourvue d'une section non filetée (33) formée par découpe.

7. Trépan selon la revendication 6, dans lequel la section non filetée (33) est soit une section plane pourvue de manière plane selon la direction longitudinale du corps de tige (30), soit une section rainurée (33a) renfoncée vers l'intérieur à une profondeur prédéterminée selon la direction longitudinale du corps de tige (30).

8. Trépan selon l'une des revendications précédentes, dans lequel le corps perforant (10) comprend en outre une section de réception (17) dans une région centrale de celui-ci pour recevoir des fragments d'os (5a) de l'os alvéolaire (5) lors de la perforation de l'os alvéolaire (5), et un trou d'évacuation (19) qui pénètre une paroi latérale correspondante en direction de l'épaisseur, communique avec la section de réception (17), et évacue les fragments d'os (5a) de l'os alvéolaire (5) logés dans la section de réception (17).

9. Trépan selon l'une des revendications précédentes, dans lequel le corps de tige (30) comprend en outre une rainure de connexion (35) à une partie terminale de celui-ci, à laquelle est connectée une pièce à main qui procure une force de rotation, et un trou traversant d'alimentation qui pénètre à l'intérieur du corps de tige (30) en direction axiale pour alimenter une solution saline à une pièce opérative lorsque le corps perforant (10) est en rotation et perfore l'os alvéolaire (5).

10. Trépan selon l'une des revendications précédentes, dans lequel le corps perforant (10) comprend en outre une unité de saisie (118) sur une paroi interne de celui-ci pour saisir un morceau d'os alvéolaire (5a) généré lors de la perforation de l'os alvéolaire (5).

11. Trépan selon la revendication 10, dans lequel l'unité de saisie (118) est une vis femelle de type rochet pour saisir les morceaux d'os alvéolaire (5a) uniquement si le corps perforant (10) tourne dans une direction.
